# EUROPÄISCHE PATENTSCHRIFT

(11) **EP 1 330 639 B1**
(45) Veröffentlichungstag und Bekanntmachung des Hinweises auf die Patenterteilung: **29.06.2005**
(21) Anmeldenummer: 01992880.3
(22) Anmeldetag: 19.10.2001
(51) Int. Cl.: G01N 24/08, G01R 33/28, A61K 9/70, A61L 33/00

(54) **MIT EINEM NMR-KONTRASTMITTEL VERSEHENER WERKSTOFF UND VERFAHREN ZU SEINER HERSTELLUNG**
MATERIAL WHICH IS PROVIDED WITH AN NMR CONTRAST AGENT AND METHOD FOR PRODUCING THE SAME
MATERIAU DOTE D'UN PRODUIT DE CONTRASTE A RESONANCE MAGNETIQUE NUCLEAIRE ET SON PROCEDE DE PRODUCTION

(30) Priorität: 03.11.2000 DE 10054667
(43) Veröffentlichungstag der Anmeldung: 30.07.2003
(73) Patentinhaber: Intech Thüringen GmbH, 99880 Waltershausen (DE)
(72) Erfinder: BLÜMICH, Bernhard, 52159 Roetgen (DE); SCHUNK, Werner, 99867 Gotha (DE); BRUDER, Michael, 22147 Hamburg (DE); KRAUSE, Karl-Heinz, 09123 Chemnitz (DE); MERKMANN, Gerhard, 99867 Gotha (DE)
(86) Internationale Anmeldenummer: PCT/DE2001/004000
(87) Internationale Veröffentlichungsnummer: WO 2002/037088

(56) Entgegenhaltungen:
- US-A- 5 277 896
- SCHUNK W ET AL: "STRAHLUNGSVERNETZTE WERKSTOFFE MIT ANTITHROMBOTISCHEN EIGENSCHAFTEN" GUMMI, FASERN, KUNSTSTOFFE. INTERNATIONALE FACHZEITSCHRIFT FUR DIE POLYMER-VERARBEITUNG, GENTNER VERLAG. STUTTGART, DE, Bd. 44, Nr. 2, 1. Februar 1991 (1991-02-01), Seiten 67-68, XP000237457 ISSN: 0176-1625
- GUTHAUSEN A ET AL: "Analysis of polymer materials by surface NMR via the MOUSE" JOURNAL OF MAGNETIC RESONANCE, ACADEMIC PRESS, ORLANDO, FL, US, Bd. 130, Nr. 1, Januar 1998 (1998-01), Seiten 1-7, XP002154163 ISSN: 1090-7807

## Beschreibung

Die Erfindung betrifft einen Werkstoff, umfassend wenigstens:
- eine Matrix auf Polymerbasis; sowie
- einen Träger, der in die Matrix eingearbeitet ist;
wobei der Werkstoff so beschaffen ist, dass mittels eines NMR-Gerätes Materialdaten zerstörungsfrei erfassbar sind.

Die Kemspin-Tomographie (MRT) arbeitet nicht mit energiereichen Röntgenstrahlen, sondern mit einem starken Magnetfeld. Atomkerne drehen sich dabei um die eigene Achse (Kemspin) und richten sich in einem Magnetfeld wie Eisenspäne alle in eine Richtung aus. Der Spin ist die Ursache für die Fähigkeit zur Magnetresonanz, denn jedes Teilchen mit Spin ist auch magnetisch. In den Atomkernen kompensieren sich die Spins der Protonen sowie Neutronen prinzipiell je paarweise. Das heißt: Kerne mit sowohl einer geraden Anzahl von Protonen als auch von Neutronen besitzen keinen resultierenden Gesamtspin. Sie sind somit magnetisch neutral. Atomkerne mit einer ungeraden Anzahl von Protonen oder Neutronen besitzen einen resultierenden Spin, den Kemspin. Das magnetische Gleichgewicht des Werkstoffes wird gestört und man erhält eine messbare magnetische Resonanz.

Ein Werkstoff des eingangs genannten Art ist in Gummi, Faser, Kunststoffe, 44 (1991) No. 2, 5.67-68 in Form eines Drainageschlauches offenbart, aus dem ein Werkstoff über einen Längeren Zeitraum abgegeben werden kann.

In der Offenlegungsschrift DE 199 39 626 A1 wird ein Verfahren zur Erzeugung von Messsignalen in Magnetfeldern, die von einem NMR-Gerät in dessen Umfeld im Umgebungsmedium erzeugt werden und deren Veränderungen zu messen sind, beschrieben. Das NMR-Gerät, das zur zerstörungsfreien Ermittlung von Materialdaten dient, ist dabei ein NMR-MOUSE-Gerät (Nuclear Magnetic Resonance MObile Universal Surface Explorer), insbesondere in Form einer NMR-MOUSE-Sonde.

In der WO 00/79253, welche einen Werkstoff des eingangs genannten Art offenbart, wird ferner beschrieben, wie mittels einer NMR-MOUSE-Sonde Flächenwaren aus polymeren Werkstoffen mit eingebetteten textilen Festigkeitsträgern die Anordnung und der Verlauf der Festigkeitsträger zerstörungsfrei und ohne Beeinträchtigung der Qualität der Flächenware feststellbar ist. Anwendbar ist dabei diese Methode für Dachbeläge, Drucktücher, Fördergurte, Membranen sowie für zylindrische Flächenwaren (Schläuche, Kompensatoren, Luftfederbälge). Von besonderer Bedeutung ist dabei die Überprüfung der Anordnung der axial verlaufenden Festigkeitsträger von Axialbälgen (DE 36 43 073 A1).

Bislang jedoch ist diese zerstörungsfreie Prüfmethode bei Werkstoffen, die eine Polymermatrix umfassen, nur begrenzt einsatzfähig.

Im Rahmen einer Weiterentwicklung besteht die Aufgabe der Erfindung darin, einen Werkstoff bereitzustellen, der bei der oben vorgestellten zerstörungsfreien Prüfmethode für eine größere Artikelpalette zur Verfügung steht.

Zwecks Lösung dieser Aufgabe zeichnet sich nun der erfindungsgemäße Werkstoff dadurch aus, dass
der Träger mit wenigstens einem Wirkstoff sowie ferner mit einem NMR-Kontrastmittel beladen ist, und zwar unter Bildung eines Träger/Wirkstoff/Kontrastmittel-Adduktes,
so dass mittels des NMR-Gerätes die Verteilung des Adduktes und somit des Wirkstoffes in der Matrix anhand des NMR-Kontrastmittels messbar wird.

Die Matrix ist insbesondere ein Polymerwerkstoff auf der Basis eines Elastomeren, thermoplastischen Elastomeren oder eines thermoplastischen Kunststoffes, wobei das Addukt innerhalb der Matrix im wesentlichen gleichmäßig verteilt ist.

Das Addukt weist vorzugsweise einen Anteil von 2 bis 30 Gew.-%, vorzugsweise 15 bis 25 Gew.-%, auf, und zwar bezogen auf die Gesamtmasse der Matrix. Die Beladungsmenge des Wirkstoffes beträgt 2 bis 20 Gew.-% und die des Kontrastmittels 1 bis 10 Gew.-%, und zwar jeweils bezogen auf die Gesamtmasse des Adduktes.

Das Kontrastmittel ist insbesondere ein Element aus der Gruppe der Seltenen Erden, wobei hier das Gadolinium (Gd) von besonderer Bedeutung ist, und zwar wiederum vorzugsweise als wässrige Lösung in Form der Gadopentetsäure.

Wenn ein unmagnetischer Werkstoff, der beispielsweise Gadolinium enthält, in ein Magnetfeld gebracht wird, so wird eine nach außen wirksame Magnetisierung des Werkstoffes in Richtung der Feldlinien gemessen, das heißt, sie nehmen die Energie des Erregers auf und werden dadurch von ihrer Ausrichtungsachse abgelenkt. Nach dem Ausschalten des Erregers (Magnetfeld) kehren sie in die ursprüngliche Position zurück und geben die aufgenommene Energie wieder ab. Diese Signale werden erfasst und werden dann zur Bewertung des Werkstoffes sowie für die Kontrolle seiner Funktionstüchtigkeit herangezogen.

Ferner ist es von Vorteil, wenn der Träger ein Molekularsieb ist, das mit dem Wirkstoff und dem Kontrastmittel ein Molekularsieb/Wirkstoff/Kontrastmittel-Addukt bildet, wobei das Molekularsieb insbesondere ein Metall-Aluminium-Silikat der folgenden Formel ist:

Meₙ [(AlO₂)_{X} · (SiO₂)_{Y}] · mH₂O

Das Molekularsieb enthält dabei eine Grundmolmenge (m) an Kristallwasser von wenigstens 100, insbesondere wiederum wenigstens 200. In diesem Zusammenhang ist insbesondere das Natrium-Aluminium-Silikat der folgenden Formel zu nennen.

Na₈₆ [(AlO₂)₈₆ · (SiO₂)₁₀₆] · 276H₂O

Im Rahmen einer besonders zweckmäßigen Modifizierung enthält das Molekularsieb/Wirkstoff/Kontrastmittel-Addukt Kristallwasser, und zwar derart, dass in Bezug auf eine ausreichende Grundmolmenge (m; z.B. m = 276) an Kristallwasser das Molekularsieb partiell dehydratisiert ist, wobei das Molekularsieb mit der reduzierten Molmenge (m'; z.B. m' = 150) mit dem Wirkstoff und dem Kontrastmittel beladen ist. Das partiell dehydratisierte Molekularsieb weist dabei einen Dehydratisierungsgrad von mindestens 20 %, vorzugsweise von 40 % bis 70 % auf. Ferner ist der Gesamtbeladungsgrad des Wirkstoffes und des Kontrastmittels im Addukt kleiner als der Dehydratisierungsgrad des partiell dehydratisierten Molekularsiebes, wobei der Beladungsgrad des Wirkstoffes mindestens 50 % und der des Kontrastmittels mindestens 10 % des Dehydratisierungsgrades beträgt.

Im folgenden werden zwei vorteilhafte Anwendungsbereiche (A, B) des neuen Werkstoffes vorgestellt.
A) Der Werkstoff (Biomaterial) wird zur Herstellung von Erzeugnissen im medizinischen Anwendungsbereich (z.B. Dialyseschläuche, Operationsschläuche, Wirkstoffpflaster) verwendet, wobei der Wirkstoff, beispielsweise ein Antithrombotikum, desorptiv freigegeben wird, während das Kontrastmittel, vorzugsweise in der oben genannten Art, aufgrund seiner ausgeprägten chemischen und physikalischen Bindungsfähigkeit am Träger verbleibt.
   Im Zusammenhang mit der Verwendung eines partiell dehydratisierten Molekularsiebes kann über den Dehydratisierungsgrad die Abgabegeschwindigkeit des Wirkstoffes gesteuert werden. So wird beispielsweise ein Addukt mit einem Dehydratisierungsgrad von 60 % begieriger Wasser aufnehmen als ein Addukt mit einem Dehydratisierungsgrad von 30 %, und zwar in Bezug auf die gleiche Beladungsmenge an Wirkstoff.
   Im medizinischen Anwendungsbereich ist es dabei besonders vorteilhaft, wenn das Addukt innerhalb der Matrix im wesentlichen gleichmäßig verteilt ist, wobei die Verteilung des Adduktes und somit des Wirkstoffes mittels des Kontrastmittels und des NMR-Gerätes messbar ist.
   Die bei der Operation eingesetzten Werkstoffe, beispielsweise Operationsschläuche, müssen zudem für weitere Kontrollen des Operationsverlaufes mittels NMR-Technik kenntlich dargestellt werden, um die Lage derartiger Schläuche gegenüber den Körperorganen exakt abzugrenzen und deren Funktion zu prüfen. Dies alles kann mit dem neuen Werkstoff erreicht werden.
   Das neue Werkstoffkonzept eröffnet zudem neue Möglichkeiten chirurgischer Eingriffe am Gehirn, an der Schädelbasis, der Wirbelsäule und der Brust. Selbstverständlich kann der Träger bzw. das Molekularsieb mit mehreren Wirkstoffen beladen werden, beispielsweise mit einem Antithrombotikum und Antibiotikum.
   In der DE-Z Schunk W. et al, "Strahlungsvemetzte Werkstoffe mit antithrombotischen Eigenschaften", Gummi, Faser, Kunststoffe, 44 (1991), No. 2, S. 67-68, werden Werkstoffe mit antithrombotischen Eigenschaften vorgestellt, und zwar insbesondere zur Verwendung von Drainageschläuchen. Im Rahmen der Schlauchherstellung wird dabei die Strahlungsvemetzung als schonende Vernetzungsmethode beschrieben.
B) Der Werkstoff wird zur Herstellung von Polymermembranen verwendet, wobei der Wirkstoff eine protonenleitende Substanz ist, beispielsweise eine Säure (z.B. Phosphorsäure).
   Auch hier spielt das partiell dehydratisierte Molekularsieb eine besondere Rolle, wobei ein Teil des Kristallwassers zwecks Erhöhung der Protonenleitfähigkeit unter Bezug auf das oben genannte Beispiel mit einer Säure beladen ist.
   Als besonderer Anwendungsfall sind hier die protonenleitenden Membranen für Brennstoffzellen zu nennen. Im Gegensatz zu dem Anwendungsbereich gemäß (A) findet hier keine Wirkstoffabgabe statt.
   Auch hier ist es von Vorteil, wenn im Hinblick auf eine optimale Wirksamkeit der Protonenleitfähigkeit der Membran das Addukt innerhalb der Matrix im wesentlichen gleichmäßig verteilt ist, wobei die Verteilung des Adduktes und somit der protonenleitenden Substanz mittels des Kontrastmittels und des NMR-Gerätes messbar ist.

Darüber hinaus besteht die Aufgabe der Erfindung darin, ein Verfahren zur Herstellung des erfindungsgemäßen Werkstoffe bereitzustellen.

Das Verfahren zur Herstellung des neuen Werkstoffes zeichnet sich nun durch folgende Verfahrensschritte aus:
- der Träger wird mit wenigstens einem Wirkstoff sowie mit dem NMR Kontrastmittel unter Bildung des Adduktes beladen;
- schließlich wird das Addukt in die Matrix eingemischt.

Dabei ist es unerheblich, in welcher Reihenfolge die Beladung des Trägers mit dem Wirkstoff und dem Kontrastmittel erfolgt.

In Verbindung mit dem Einsatz des partiell dehydratisierten Molekularsiebes gelten folgende Verfahrensschritte:
- das Molekularsieb mit einer ausreichenden Grundmolmenge (m) wird bei 300 bis 500°C, vorzugsweise bei 400 bis 450°C, mehrere Stunden, vorzugsweise 2 bis 6 Stunden, lang partiell dehydratisiert;
- anschließend wird das partiell dehydratisierte Molekularsieb mit der reduzierten Molmenge (m') an Kristallwasser mit wenigstens einem Wirkstoff sowie mit dem Kontrastmittel unter Bildung des Adduktes beladen;
- schließlich wird das Addukt in die Matrix eingemischt.

Die Dehydratisierung und/oder Beladung wird/werden dabei insbesondere in Gegenwart eines inerten Gases, beispielsweise von Stickstoff, durchgeführt. Die Dehydratisierung und/oder Beladung erfolgt/erfolgen ferner vorzugsweise unter Normaldruck. Die Beladung selbst kann beispielsweise mittels einer Kugelmühle vorgenommen werden.

Die Erfindung wird nun anhand eines Ausführungsbeispieles im medizinischen Anwendungsbereich unter Bezugnahme auf eine schematische Zeichnung erläutert.

In Form eines einfachen Ausführungsbeispieles umfasst das medizinische Erzeugnis **1** eine Matrix **2,** die dem spezifischen Anwendungsbereich entsprechend geformt ist.

In die Matrix **2** ist nun das Molekularsieb/Wirkstoff/Kontrastmittel-Addukt **3** eingemischt, wobei das Molekularsieb partiell dehydratisiert ist.

Im Rahmen einer Qualitätskontrolle kann nun mittels des Kontrastmittels X und des NMR-Gerätes **5,** vorzugsweise in Form einer NMR-MOUSE-Sonde, die optimale Verteilung des Adduktes **3** und somit des Wirkstoffes Z innerhalb der Matrix **2** ermittelt werden (Abbildung ohne medizinisch wirksame Kontaktzone **4**). Der Werkstoff ist dabei im oberflächennahen Bereich des NMR-Gerätes angeordnet.

Bei Kontakt **4** mit der wässrigen Körperflüssigkeit findet nun eine Adsorption von Wasser unter Desorption des Wirkstoffes Z statt, während das Kontrastmittel X in der Matrix **2** verbleibt.

Ist beispielsweise die Matrix **2** mit dem eingemischten Addukt **3** die Wand eines Dialyseschlauches, so wird der Wirkstoff Z vom Blut aufgenommen.

Mit der NMR-Technik, wie sie in den beiden Offenlegungsschriften DE 199 28 039 A1 und DE 199 39 626 A1 ausführlich beschrieben wird, kann man bei dem erfindungsgemäßen Werkstoff zudem den Verlauf der Wirkstoffabgabe beobachten.

### Bezugszeichenliste

- **1**: Erzeugnis für den medizinischen Anwendungsbereich
- **2**: Matrix
- **3**: Träger/Wirkstoff/Kontrastmittel-Addukt Molekularsieb/Wirkstoff/Kontrastmittel-Addukt
- **4**: Kontaktzone mit der wässrigen Körperflüssigkeit (Haut, Körpergewebe, Blut)
- **5**: NMR-Gerät (NMR-MOUSE-Sonde)
- X: Kontrastmittel
- Z: Wirkstoff

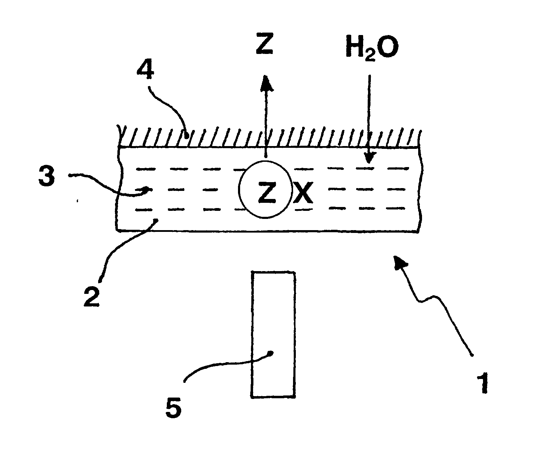

## Patentansprüche

1. Werkstoff, umfassend wenigstens:
- eine Matrix (2) auf Polymerbasis; sowie
- einen Träger, der in die Matrix eingearbeitet ist;
wobei der Werkstoff so beschaffen ist, dass mittels eines NMR-Gerätes (5) Materialdaten zerstörungsfrei erfassbar sind,
wobei der Träger mit wenigstens einem Wirkstoff (Z) sowie ferner mit einem NMR-Kontrastmittel (X) beladen ist, und zwar unter Bildung eines Träger/Wirkstoff/Kontrastmittel-Adduktes (3),
so dass mittels des NMR-Gerätes (5) die Verteilung des Adduktes (3) und somit des Wirkstoffes (Z) in der Matrix (2) anhand des NMR-kontrastmittels (X) messbar wird.

2. Werkstoff nach Anspruch 1, **dadurch gekennzeichnet, dass** die Matrix (2) ein Polymerwerkstoff auf der Basis eines Elastomeren, thermoplastischen Elastomeren oder eines thermoplastischen Kunststoffes ist.

3. Werkstoff nach Anspruch 1 oder 2, **dadurch gekennzeichnet, dass** das Addukt (3) innerhalb der Matrix (2) im wesentlichen gleichmäßig verteilt ist.

4. Werkstoff nach einem der Ansprüche 1 bis 3, **dadurch gekennzeichnet, dass** das Addukt (3) einen Anteil von 2 bis 30 Gew.-%, vorzugsweise 15 bis 25 Gew.-%, aufweist, und zwar bezogen auf die Gesamtmasse der Matrix (2).

5. Werkstoff nach einem der Ansprüche 1 bis 4, **dadurch gekennzeichnet, dass** die Beladungsmenge des Wirkstoffes (Z) 2 bis 20 Gew.-% beträgt, und zwar bezogen auf die Gesamtmasse des Adduktes (3).

6. Werkstoff nach einem der Ansprüche 1 bis 5, **dadurch gekennzeichnet, dass** die Beladungsmenge des Kontrastmittels (X) 1 bis 10 Gew.-% beträgt, und zwar bezogen auf die Gesamtmasse des Adduktes (3).

7. Werkstoff nach einem der Ansprüche 1 bis 6, **dadurch gekennzeichnet, dass** das Kontrastmittel (X) ein Element aus der Gruppe der Seltenen Erden ist.

8. Werkstoff nach Anspruch 7, **dadurch gekennzeichnet, dass** das Kontrastmittel (X) Gadolinium ist.

9. Werkstoff nach Anspruch 8, **dadurch gekennzeichnet, dass** das Gadolinium als wässrige Lösung in Form der Gadopentetsäure vorliegt.

10. Werkstoff nach einem der Ansprüche 1 bis 9, **dadurch gekennzeichnet, dass** der Träger ein Molekularsieb ist, das mit dem Wirkstoff (Z) und dem Kontrastmittel (X) ein Molekularsieb/Wirkstoff/Kontrastmittel-Addukt (3) bildet.

11. Werkstoff nach Anspruch 10, **dadurch gekennzeichnet, dass** das Molekularsieb ein Metall-Aluminium-Silikat der folgenden Formel ist:
Meₙ [(AlO₂)_{X} · (SiO₂)_{Y}] · mH₂O

12. Werkstoff nach Anspruch 11, **dadurch gekennzeichnet, dass** ein Metall der ersten oder zweiten Hauptgruppe des Periodensystems, vorzugsweise Natrium, Verwendung findet.

13. Werkstoff nach Anspruch 11 oder 12, **dadurch gekennzeichnet, dass** das Molekularsieb ein Natrium-Aluminium-Silikat der folgenden Formel ist:
Na₈₆ [(AlO₂)₈₆ · (SiO₂)₁₀₆] · mH₂O

14. Werkstoff nach einem der Ansprüche 10 bis 13, **dadurch gekennzeichnet, dass** das Molekularsieb eine Grundmolmenge (m) an Kristallwasser von wenigstens 100, insbesondere wenigstens 200, enthält.

15. Werkstoff nach Anspruch 14, **dadurch gekennzeichnet, dass** die Grundmolmenge (m) an Kristallwasser 276 beträgt.

16. Werkstoff nach Anspruch 13 und 15, **dadurch gekennzeichnet, dass** das Molekularsieb ein Natrium-Aluminium-Silikat der folgenden Formel ist:
Na₈₆ [(AlO₂)₈₆ · (SiO₂)₁₀₆] · 276 H₂O

17. Werkstoff nach einem der Ansprüche 10 bis 16, **dadurch gekennzeichnet, dass** das Molekularsieb/Wirkstoff/Kontrastmittel-Addukt (3) Kristallwasser enthält, und zwar derart, dass in Bezug auf eine ausreichende Grundmolmenge (m) an Kristallwasser das Molekularsieb partiell dehydratisiert ist, wobei das Molekularsieb mit der reduzierten Molmenge (m') an Kristallwasser mit dem Wirkstoff (Z) und dem Kontrastmittel (X) beladen ist.

18. Werkstoff nach Anspruch 17, **dadurch gekennzeichnet, dass** das partiell dehydratisierte Molekularsieb einen Dehydratisierungsgrad von mindestens 20 %, vorzugsweise von 40 % bis 70 %, aufweist.

19. Werkstoff nach Anspruch 17 oder 18, **dadurch gekennzeichnet, dass** der Gesamtbeladungsgrad des Wirkstoffes (Z) und des Kontrastmittels (X) im Addukt (3) kleiner ist als der Dehydratisierungsgrad des partiell dehydratisierten Molekularsiebes.

20. Werkstoff nach Anspruch 19, **dadurch gekennzeichnet, dass** der Beladungsgrad des Wirkstoffes (Z) mindestens 50 % des Dehydratisierungsgrades beträgt.

21. Werkstoff nach Anspruch 19 oder 20, **dadurch gekennzeichnet, dass** der Beladungsgrad des Kontrastmittels (X) mindestens 10 % des Dehydratisierungsgrades beträgt.

22. Verwendung des Werkstoffes nach einem der Ansprüche 1 bis 21 zur Herstellung von Erzeugnissen im medizinischen Anwendungsbereich, wobei der Wirkstoff (Z) desorptiv freigegeben wird, während das Kontrastmittel (X) am Träger gebunden bleibt.

23. Verwendung des Werkstoffes nach einem der Ansprüche 1 bis 21, zur Herstellung von Membranen, wobei der Wirkstoff (Z) eine protonenleitende Substanz ist.

24. Verfahren zur Herstellung eines Werkstoffes nach einem der Ansprüche 1 bis 23, **gekennzeichnet durch** folgende Verfahrensschritte:
- der Träger wird mit wenigstens einem Wirkstoff (Z) sowie mit dem NMR-Kontrastmittel (X) unter Bildung des Adduktes (3) beladen;
- schließlich wird das Addukt (3) in die Matrix (2) eingemischt.

25. Verfahren zur Herstellung eines Werkstoffes nach Anspruch 24 in Verbindung mit einem der Ansprüche 17 bis 21, **gekennzeichnet dadurch, dass** vor des beladung des Trägers in Form des Molekularsiebs
das Molekularsieb mit einer ausreichenden Grundmolmenge (m) an kristallwasser bei 300 bis 500°C, vorzugsweise bei 400 bis 450°C, mehrere Stunden, vorzugsweise 2 bis 6 Stunden, lang partiell dehydratisiert wird.

26. Verfahren zur Herstellung eines Werkstoffes nach Anspruch 25, **dadurch gekennzeichnet, dass** die Dehydratisierung und/oder Beladung in Gegenwart eines inerten Gases, beispielsweise von Stickstoff, durchgeführt wird/werden.

27. Verfahren zur Herstellung eines Werkstoffes nach einem der Ansprüche 25 oder 26, **dadurch gekennzeichnet, dass** die Dehydratisierung und/oder Beladung unter Normaldruck durchgeführt wird/werden.

## Claims

1. Material comprising at least:
- a polymer-based matrix (2) and
- a carrier which is incorporated in the matrix,
the material being such that material data can be detected without destruction using an NMR device (5),
the carrier being charged with at least one active substance (Z) and with an NMR contrast medium (X) so as to form a carrier/active substance/contrast medium adduct (3),
so that the distribution of the adduct (3) and hence the active substance (Z) in the matrix (2) can be measured by means of the NMR contrast medium (X) using the NMR device (5).

2. Material according to claim 1, **characterised in that** the matrix (2) is a polymer material based on an elastomer, thermoplastic elastomer or thermoplastic plastic.

3. Material according to claim 1 or 2, **characterised in that** the adduct (3) is distributed essentially uniformly within the matrix (2).

4. Material according to one of claims 1 to 3, **characterised in that** the adduct (3) makes up 2 to 30% by weight, preferably 15 to 25% by weight, related to the total mass of the matrix (2).

5. Material according to one of claims 1 to 4, **characterised in that** the charge of the active substance (Z) is 2 to 20% by weight, related to the total mass of the adduct (3).

6. Material according to one of claims 1 to 5, **characterised in that** the charge of the contrast medium (X) is 1 to 10% by weight, related to the total mass of the adduct (3).

7. Material according to one of claims 1 to 6, **characterised in that** the contrast medium (X) is an element from the group of rare earths.

8. Material according to claim 7, **characterised in that** the contrast medium (X) is gadolinium.

9. Material according to claim 8, **characterised in that** the gadolinium is present as an aqueous solution in the form of gadopentetic acid.

10. Material according to one of claims 1 to 9, **characterised in that** the carrier is a molecular sieve which forms a molecular sieve/active substance/contrast medium adduct (3) with the active substance (Z) and the contrast medium (X).

11. Material according to claim 10, **characterised in that** the molecular sieve is a metal aluminium silicate with the following formula:
Meₙ [(AlO₂)_{X} · (SiO₂)_{Y}] · mH₂O

12. Material according to claim 11, **characterised in that** a metal of the first or second main group of the periodic system, preferably sodium, is used.

13. Material according to claim 11 or 12, **characterised in that** the molecular sieve is a sodium aluminium silicate with the following formula:
Na₈₆ [(AlO₂)₈₆ · (SiO₂)₁₀₆] · mH₂O

14. Material according to one of claims 10 to 13, **characterised in that** the molecular sieve contains a basic molar amount (m) of water of crystallisation of at least 100, in particular at least 200.

15. Material according to claim 14, **characterised in that** the basic molar amount (m) of water of crystallisation is 276.

16. Material according to claim 13 and 15, **characterised in that** the molecular sieve is a sodium aluminium silicate with the following formula:
Na₈₆ [(AlO₂)₈₆ · (SiO₂)₁₀₆] · 276H₂O

17. Material according to one of claims 10 to 16, **characterised in that** the molecular sieve/active substance/contrast medium adduct (3) contains water of crystallisation, such that the molecular sieve is partially dehydrated in terms of a sufficient basic molar amount (m) of water of crystallisation, the molecular sieve with the reduced molar amount (m') of water of crystallisation being charged with the active substance (Z) and the contrast medium (X).

18. Material according to claim 17, **characterised in that** the partially dehydrated molecular sieve exhibits a degree of dehydration of at least 20%, preferably 40% to 70%.

19. Material according to claim 17 or 18, **characterised in that** the total degree of charging of the active substance (Z) and the contrast medium (X) in the adduct (3) is less than the degree of dehydration of the partially dehydrated molecular sieve.

20. Material according to claim 19, **characterised in that** the degree of charging of the active substance (Z) is at least 50% of the degree of dehydration.

21. Material according to claim 19 or 20, **characterised in that** the degree of charging of the contrast medium (X) is at least 10% of the degree of dehydration.

22. Use of the material according to one of claims 1 to 21 for manufacturing products in the medical field of application, in which the active substance (Z) is released desorptively while the contrast medium (X) remains bound to the carrier.

23. Use of the material according to one of claims 1 to 21 for manufacturing membranes, in which the active substance (Z) is a proton-conducting substance.

24. Method for manufacturing a material according to one of claims 1 to 23, **characterised by** the following process steps:
- the carrier is charged with at least one active substance (Z) and with the NMR contrast medium (X) so as to form the adduct (3);
- finally the adduct (3) is mixed into the matrix (2).

25. Method for manufacturing a material according to claim 24 in conjunction with one of claims 17 to 21, **characterised in that** before the charging of the carrier in the form of the molecular sieve, the molecular sieve with a sufficient basic molar amount (m) of water of crystallisation is partially dehydrated at 300 to 500°C, preferably at 400 to 450°C, for a plurality of hours, preferably 2 to 6 hours.

26. Method for manufacturing a material according to claim 25, **characterised in that** the dehydration and/or charging is/are carried out in the presence of an inert gas, for example nitrogen.

27. Method for manufacturing a material according to one of claims 25 or 26, **characterised in that** the dehydration and/or charging is/are carried out under normal pressure.

## Revendications

1. Matériau comprenant au moins :
- une matrice (2) à base d'un polymère ; et
- un support, qui est incorporé dans la matrice,
le matériau étant proposé de manière qu'à l'aide d'un appareil RMN (5), les caractéristiques du matériau sont détectables d'une manière non destructive, le support étant chargé d'au moins une matière active (Z), ainsi que d'un produit de contraste (X) pour RMN, et plus précisément avec formation d'un produit d'addition (3) support/matière active/produit de contraste, de telle sorte qu'il soit possible, à l'aide de l'appareil RMN (5) de mesurer la répartition du produit d'addition (3), et donc de la matière active (Z) dans la matrice (2) à l'aide du produit de contraste (X) pour RMN.

2. Matériau selon la revendication 1, **caractérisé en ce que** la matrice (2) est un matériau polymère à base d'un élastomère, d'un élastomère thermoplastique ou d'une matière plastique thermoplastique.

3. Matériau selon la revendication 1 ou 2, **caractérisé en ce que** le produit d'addition (3) est réparti pour l'essentiel uniformément à l'intérieur de la matrice (2).

4. Matériau selon l'une des revendications 1 à 3, **caractérisé en ce que** le produit d'addition (3) représente 2 à 30 % en poids et de préférence 15 à 25 % en poids par rapport au poids total de la matrice (2).

5. Matériau selon l'une des revendications 1 à 4, **caractérisé en ce que** la quantité de charge de la matière active (Z) est de 2 à 20 % en poids par rapport au poids total du produit d'addition (3).

6. Matériau selon l'une des revendications 1 à 5, **caractérisé en ce que** la quantité de charge du produit de contraste (X) est de 1 à 10 % en poids par rapport au poids total du produit d'addition (3).

7. Matériau selon l'une des revendications 1 à 6, **caractérisé en ce que** le produit de contraste (X) est un élément du groupe des terres rares.

8. Matériau selon la revendication 7, **caractérisé en ce que** le produit de contraste (X) est le gadolinium.

9. Matériau selon la revendication 8, **caractérisé en ce que** le gadolinium se présente en solution aqueuse sous forme de l'acide gadopentétique.

10. Matériau selon l'une des revendications 1 à 9, **caractérisé en ce que** le support est un tamis moléculaire, qui avec la matière active (Z) et le produit de contraste (X) forme un produit d'addition (3) tamis moléculaire/matière active/produit de contraste.

11. Matériau selon la revendication 10, **caractérisé en ce que** le tamis moléculaire est un aluminosilicate métallique ayant la formule suivante :
Meₙ[(AlO₂)ₓ.(SiO₂)_{y}].mH₂O

12. Matériau selon la revendication 11, **caractérisé en ce qu'**on y utilise un métal du premier ou du deuxième groupe principal du Tableau Périodique, de préférence le sodium.

13. Matériau selon la revendication 11 ou 12, **caractérisé en ce que** le tamis moléculaire est un aluminosilicate de sodium ayant la formule suivante :
Na₈₆[(AlO₂)₈₆.(SiO₂)₁₀₆].mH₂O

14. Matériau selon l'une des revendications 10 à 13, **caractérisé en ce que** le tamis moléculaire contient une quantité molaire de base (m) d'eau de cristallisation d'au moins 100 et en particulier d'au moins 200.

15. Matériau selon la revendication 14, **caractérisé en ce que** la quantité molaire de base (m) d'eau de cristallisation est de 276.

16. Matériau selon les revendications 13 et 15, **caractérisé en ce que** le tamis moléculaire est un aluminosilicate de sodium ayant la formule suivante :
Na₈₆[(AlO₂)₈₆.(SiO₂)₁₀₆].276H₂O

17. Matériau selon l'une des revendications 10 à 16, **caractérisé en ce que** le produit d'addition (3) tamis moléculaire/matière active/produit de contraste contient de l'eau de cristallisation de telle sorte que, par rapport à une quantité molaire de base suffisante (m) d'eau de cristallisation, le tamis moléculaire subisse une déshydratation partielle ; le tamis moléculaire, contenant la quantité molaire réduite (m') d'eau de cristallisation, étant chargé de la matière active (Z) et du produit de contraste (X).

18. Matériau selon la revendication 17, **caractérisé en ce que** le tamis moléculaire partiellement déshydraté présente un degré de déshydratation d'au moins 20 % et de préférence de 40 à 70 %.

19. Matériau selon la revendication 17 ou 18, **caractérisé en ce que** le degré total de charge de la matière active (Z) et du produit de contraste (X) dans le produit d'addition (3) est inférieur au degré de déshydratation du tamis moléculaire partiellement déshydraté.

20. Matériau selon la revendication 19, **caractérisé en ce que** le degré de charge de la matière active (Z) est d'au moins 50 % du degré de déshydratation.

21. Matériau selon la revendication 19 ou 20, **caractérisé en ce que** le degré de charge du produit de contraste (X) est d'au moins 10 % du degré de déshydratation.

22. Utilisation du matériau selon l'une des revendications 1 à 21 pour fabriquer des produits dans le domaine d'application médicale, la matière active (Z) étant libérée par désorption, tandis que le produit de contraste (X) reste lié au support.

23. Utilisation du matériau selon l'une des revendications 1 à 21 pour fabriquer des membranes, la matière active (Z) étant une substance conductrice de protons.

24. Procédé de fabrication d'un matériau selon l'une des revendications 1 à 23, **caractérisé par** les étapes suivantes :
- on charge le support d'au moins une matière active (Z) et du produit de contraste (X) pour RMN avec formation du produit d'addition (3) ;
- on introduit ensuite par mélange le produit d'addition (3) dans la matrice (2).

25. Procédé de fabrication d'un matériau selon la revendication 24 en liaison avec l'une des revendications 17 à 23, **caractérisé en ce que**, avant la charge du support sous forme du tamis moléculaire, le tamis moléculaire, ayant une quantité molaire de base (m) suffisante d'eau de cristallisation, est soumis à une déshydratation partielle à une température de 300 à 500°C et de préférence de 400 à 450°C pendant plusieurs heures, de préférence 2 à 6 heures.

26. Procédé de fabrication d'un matériau selon la revendication 25, **caractérisé en ce que** la déshydratation et/ou la charge sont réalisées en présence d'un gaz inerte, par exemple l'azote.

27. Procédé de fabrication d'un matériau selon l'une des revendications 25 ou 26, **caractérisé en ce que** la déshydratation et/ou la charge sont réalisées sous pression normale.
